# EUROPEAN PATENT SPECIFICATION

(11) **EP 2 685 231 B1**
(45) Date of publication and mention of the grant of the patent: **06.06.2018**
(21) Application number: 12754727.1
(22) Date of filing: 06.03.2012
(51) Int. Cl.: A61B 5/145, G01N 1/22, G01N 1/02, G01N 33/497, G01N 35/00, A61B 5/08

(54) **BODY WORN DEVICE AND METHOD FOR MEASURING BIOLOGICAL GAS**
AM KÖRPER GETRAGENE VORRICHTUNG SOWIE VERFAHREN ZUR MESSUNG VON BIOLOGISCHEM GAS
DISPOSITIF PORTÉ SUR LE CORPS ET PROCÉDÉ POUR MESURER UN GAZ BIOLOGIQUE

(30) Priority: 08.03.2011 JP 2011050744
(43) Date of publication of application: 15.01.2014
(73) Proprietor: NTT Docomo, Inc., Tokyo 100-6150 (JP)
(72) Inventor: MORITANI, Yuki, Tokyo 100-6150 (JP); HIYAMA, Satoshi, Tokyo 100-6150 (JP)
(74) Representative: Hargreaves, Timothy Edward
(86) International application number: PCT/JP2012/055715
(87) International publication number: WO 2012/121260

(56) References cited:
- JP-A- 6 000 173
- JP-A- 2004 258 761
- JP-A- 2004 294 328
- JP-A- 2009 175 111
- JP-A- 2009 257 772
- JP-A- 2010 148 692
- US-A- 5 314 389
- US-A1- 2006 058 697
- US-A1- 2007 083 095
- US-A1- 2007 276 270
- US-A1- 2009 227 887

## Description

### TECHNICAL FIELD

The present invention relates to a biological gas measurement device and method and more particularly relates to a wearable device and a method for measuring a biological gas released from a skin surface or expiration of a living body.

### BACKGROUND ART

Conventionally, some techniques have been discussed for obtaining and grasping biological information, typically health condition of a living body, by measuring and analyzing gas components released from the living body. For example, Patent Document 1 (JP 2001-349888) discloses a technique for measuring the degree of combustion of body fat by detecting a concentration of acetone included in expiration gas. Also, Patent Document 2 (JP 2006-75447) discloses a technique for detecting abnormal proliferation of intestinal anaerobic bacteria and malabsorption syndrome by sensing hydrogen included in expiration gas. Also, Patent Document 3 (JP 2009-257772) and Patent Document 4 (JP 2010-26746) disclose techniques for implementing complicated medical examination by detecting several types of gas components in expiration gas by means of multiple types of gas detection elements for acetone, nitric monoxide, carbon dioxide, hydrogen and ammonia as well as detecting a single gas component in the expiration gas.

Meanwhile, some techniques have been discussed for obtaining and grasping the biological information by detecting percutaneous gas released through a skin instead of the expiration gas so that continuous measurement can be carried out without user's need of exhaling expiration gas in a detection device. For example, Patent Document 5 (JP 2006-234845) discloses a technique for sensing a user's health condition by measuring skin penetrable gas components such as acetone and hydrogen, that is, percutaneous gas, released from a part of a living body such as a finger or a palm with a wearable device worn on that part. Also, Patent Document 6 (JP 2010-148692) discloses an umbilical belt type of skin gas detection device that can monitor health condition continuously by measuring a concentration of hydrogen released from a skin surface of a living body.

### SUMMARY OF INVENTION

### [PROBLEM TO BE SOLVED BY THE INVENTION]

However, the above-stated expiration gas measurement techniques are directed to detect components in the expiration gas, and accordingly there is a problem that a user must bother to carry a dedicated expiration gas detection device for detection.

Also, since the above-stated percutaneous gas measurement techniques force the user to wear the gas measurement device, which is unnecessary and unnatural in daily life, so as to measure the percutaneous gas, and accordingly there is a problem that a significant burden is imposed on the user.

In order to overcome the above-stated problems, one object of the present invention is to provide a technique for obtaining and grasping biological information, typically a user's health condition, in a natural manner in daily life by measuring a gas component released from a living body while lessening the burden to the user.

We acknowledge the disclosure in US2007/0083095A of a watch for measuring biological gas in the space next to the skin, during exercise, and providing output information.

US2007/0276270A discloses an earphone with wireless voice and data connectivity and physiological sensors for stroke monitoring.

US2006/0058697A discloses a mobile phone with an expiration (exhalation) analysis sensor for biological gas, and an information display for biological measurement results.

### [MEANS FOR SOLVING THE PROBLEM]

In order to address the above-stated problems, the present invention is in the system of Claim 1 and the method of Claim 8.

### [ADVANTAGE OF THE INVENTION]

According to the present invention, when a user views and listens to music, video or the like, the user can measure a biological gas component released from a skin as well as viewing and listening to the music, the video or the like while exhibiting fashion sense by wearing the wearable device together with accessories. As a result, the user can obtain and grasp biological information, typically a user's own health condition, in a natural manner in daily life while reducing a user's burden involved in the wearing of a biological gas measurement device.

### BRIEF DESCRIPTION OF DRAWINGS

FIG. 1 is a schematic diagram for illustrating a biological gas measurement device according to one embodiment of the present invention; and
FIG. 2 is a schematic diagram for illustrating a biological gas measurement device according to another embodiment of the present invention.

### EMBODIMENTS FOR CARRYING OUT THE INVENTION

Embodiments of the present invention are described below with reference to the drawings.

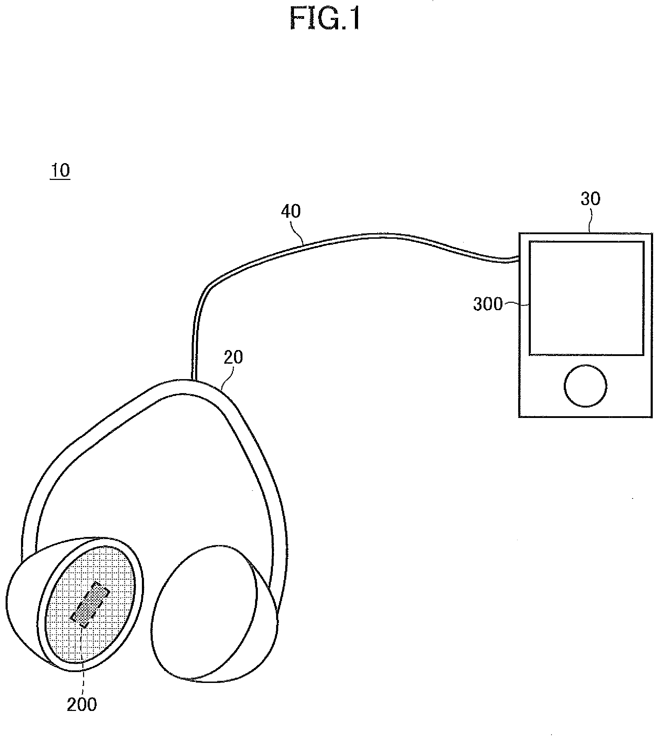

In the embodiments, as one typical example of a wearable device, a wearable sound wave conversion
device is focused on, and a biological gas measurement device according to one embodiment of the present invention is described with reference to FIG. 1. FIG. 1 is a schematic diagram for illustrating a biological gas measurement device according to one embodiment of the present invention.

As illustrated in FIG. 1, a biological gas measurement device 10 according to this embodiment includes a wearable sound wave conversion device 20, a handheld terminal 30 and a cable 40. The wearable sound wave conversion device 20 is communicatively connected to the handheld terminal 30 via the cable 40.

The wearable sound wave conversion device 20 is a wearable sound output device, typically a headphone, for providing a user with the sound via speakers closely positioned to ears when the user wears it. The wearable sound wave conversion device 20 has a sound wave conversion function to convert an electrical signal representing audio information transmitted from the handheld terminal 30 into listenable sound waves (sound).

The wearable sound wave conversion device 20 according to this embodiment has a gas reservoir structure 50 for forming a space to accumulate or accommodate a percutaneous gas component released from a skin surface around an ear of the user. Specifically, when the user wears the wearable sound wave conversion device 20, the skin around a user's ear contacts the wearable sound wave conversion device 20, which results in a closed space around a user's ear. The percutaneous gas released from the skin surface of a user's ear is accumulated or accommodated in the closed space. Although it is desirable that the space formed between the skin around a user's ear and the wearable sound wave conversion device 20 in the user wearing the wearable sound wave conversion device 20 is a closed space, it may not be a completely closed space. In other words, the space may be any appropriate space for enabling the percutaneous gas released from the ear surface to be accumulated. Also, the wearable sound wave conversion device 20 may have some structure to enable the percutaneous gas to be evacuated after a certain degree of accumulation.

Note that there is a likelihood that a moisture may arise in the formed space including the user's skin surface due to sudation and moisture occurrence from the user's skin surface, and the effects of temperature and humidity or others. Particularly in the case where a percutaneous gas component of interest is soluble, there is a risk that the concentration of the percutaneous gas cannot be correctly measured due to the effect of the moisture. To this end, according to this embodiment, the wearable sound wave conversion device 20 may further include a moisture removal and restraint mechanism 100 for removing or restraining the moisture from/in the space formed with the gas reservoir structure 50 including a user's skin surface. Specifically, the moisture removal and restraint mechanism 100 is arranged to have a water absorbent, a dehumidification agent, a desiccant agent, a miniaturized air blower or a combination thereof. Examples of the water absorbent, the dehumidification agent and the desiccant agent for removing occurring moisture includes, but is not limited to, a sodium polyacrylate, a silica gel, a calcium oxide, a calcium chloride, an activated carbon, a paper piece, a fiber and so on. Also, for example, the miniaturized air blower may blow the air on a skin surface to dry the skin surface so that the moisture may be difficult to occur.

Also, the moisture removal and restraint mechanism 100 may be activated at any appropriate timing in synchronization with measurement timings of a gas sensor device 200. Specifically, the moisture removal and restraint mechanism 100 may remove or restrain the moisture from/in the space formed with the gas reservoir structure 50 before or at the same time as the measurement timings of a percutaneous gas component by the gas sensor device 200. Furthermore, the moisture removal and restraint mechanism 100 may be activated at any appropriate timing depending on its implementations. For example, if the moisture removal and restraint mechanism 100 is implemented in the above-stated humidity absorbent such as the water absorbent, the dehumidification agent or the desiccant agent, the moisture removal and restraint mechanism 100 may be activated before or at the same time as the measurement timings of a percutaneous gas component by the gas sensor device 200. Also, if the moisture removal and restraint mechanism 100 is implemented in the miniaturized air blower, the moisture removal and restraint mechanism 100 may be activated before the measurement timings of a percutaneous gas component by the gas sensor device 200.

The wearable sound wave conversion device 20 according to this embodiment includes the gas sensor device 200 for measuring a percutaneous gas component released from the ear surface. The gas sensor device 200 is fixed to the wearable sound wave conversion device 20 to measure a percutaneous gas component accumulated in the percutaneous gas reservoir space formed when a user wears the wearable sound wave conversion device 20. The gas sensor device 200 is made of a semiconductor sensor, for example, and is arranged to have one or more sensors for measuring various gas components arising from a living body, such as acetone, hydrogen, carbon monoxide, methane, hydrogen sulfide, isoprene, trimethylamine, ammonia, methanol, acetaldehyde and ethanol, in the percutaneous gas accumulated in the formed percutaneous gas reservoir space. Note that the gas components to be measured are not limited to the above-stated gas components and may be any type of gas components included in the percutaneous gas arising from the living body. Also, the gas sensor device 200 is not limited to the semiconductor sensor and may be a carbon nanotube type sensor as disclosed in Patent Document 3 (JP 2009-257772), for example. Also, instead of these gas sensors, the gas sensor device 200 may be a graphene type sensor, an electrochemical sensor, an optical fiber type sensor, a thin-film type sensor, a MEMS thermal conducting sensor, a surface acoustic wave sensor, a micro thermal conduction-type sensor, a contact burning-type sensor, an electromotive force-type sensor and any other appropriate sensor that can measure a percutaneous gas component.

Also, the gas sensor device 200 is not limited to a sensor for measuring a certain single type of component and may be arranged to have an array of different types of sensors so that the gas sensor device 200 can measure different types of gas components simultaneously. In this case, the different sensors in the gas sensor device 200 are not limited to such an array arrangement and may be able to measure the different gas components by using a miniaturized gas-chromatographic chip, as disclosed in Non-Patent Document 1 (Leandro Lorenzelli, et al. "Development of a gas chromatography silicon-based microsystem in clinical diagnostics", Biosensors and Bioelectronics, vol. 20, pp. 1968-1976, 2005) to separate the gas components.

Also in FIG. 1, the gas sensor device 200 is fixed inside a protection mesh (a mesh for protecting a portion having the sound wave conversion function from the outside) of the wearable sound wave conversion device 20. However, the fixing position of the gas sensor device 200 is not limited to this position and may be any position where the gas component accumulated in the percutaneous gas reservoir space formed between the wearable sound wave conversion device 20 and the skin around the ear can be measured. For example, the gas sensor device 200 may be embedded in a position unobservable from the external appearance of the wearable sound wave conversion device 20.

The handheld terminal 30 is typically implemented in a mobile phone or an audio player and has an audio reproduction function to reproduce audio information such as music recorded in a storage device in the handheld terminal 30. The handheld terminal 30 outputs electrical signals representative of audio data for conversion into sound waves at the wearable sound wave conversion device 20 and transmits the electrical signals to the wearable sound wave conversion device 20 via the cable 40. Also, upon receiving a measurement result by the gas sensor device 200 via the cable 40, the handheld terminal 30 performs some operation as needed, displays the measurement result of the concentration or others of a gas component of interest on a display 300 and records the measurement result in the storage device in the handheld terminal 30. Also, if the handheld terminal 30 has a communication function like a mobile phone and so on, the measurement result may be transmitted to a server on a network or others and stored therein.

In this embodiment, the wearable sound wave conversion device 20 is connected to the handheld terminal 30 via the cable 40. In another embodiment, signals may be communicated between the wearable sound wave conversion device 20 and the handheld terminal 30 wirelessly rather than the cable 40. Also, in the case of a cable or wired connection, multiple cables may be used to prevent interference between electrical signals such as music and electrical signals of measurement results and/or to separate the respective channels physically. In other words, any communication implementation that can transmit and receive signals between the handheld terminal 30 and the wearable sound wave conversion device 20 may be used. Also, as long as information reproduced by the handheld terminal 30 includes information converted by the wearable sound wave conversion device 20 into sound waves, the reproduced information is not limited to music or others and may be composite information of video information and audio information. In this case, the video information is displayed on the display 300, and the audio information is supplied to the wearable sound wave conversion device 20 in synchronization with the video information displayed on the display 300 similar to music or others.

As stated above, according to the biological gas measurement device 10 of this embodiment, when a user wears the wearable sound wave conversion device 20 to view and listen to music, a video and so on recorded in the handheld terminal 30, the percutaneous gas reservoir space is formed around a user's ears. Since a percutaneous gas released from a user's skin is accumulated in the formed percutaneous gas reservoir space, the gas sensor device 200 can measure a gas component such as acetone in the percutaneous gas. This measurement result is conveyed to the handheld terminal 30 via the cable 40, is displayed on the display 300 and/or recorded in the handheld terminal 30 and/or an external server, whereby the user can know various biological information such as the concentration of acetone in a user's own percutaneous gas.

Also, the handheld terminal 30 may analyze the measured various percutaneous gas components and provide the user with appropriate guide information. For example, an exemplary case where the user is walking with the wearable sound wave conversion device 20 in order to lose weight is considered below. In this case, when the measured concentration of acetone released from the user has not reached such a degree of concentration that a weight reduction effect can be obtained, the handheld terminal 30 may provide the user with guide information to urge the user to increase the walking speed. Thereby, the user can achieve more effective exercise outcomes.

Also, a user may not only recognize the biological information and the guide information based on the measurement result as image information and/or text information on the display 300 but also obtain audio information from the wearable sound wave conversion device 20. Specifically, the biological information and the guide information based on the measurement result may be converted into audio information, and the converted audio information may be superimposed to other audio information such as music supplied from the wearable sound wave conversion device 20 so that the combined audio information may be provided to the user. Thereby, the user can obtain the biological information and the guide information from the wearable sound wave conversion device 20 without viewing the display 300.

Although it has been described that the gas sensor device 200 of this embodiment is fixed to one side of an attachment portion of the wearable sound wave conversion device 20, the gas sensor device 200 may be fixed to both sides. The attachment of the gas sensor device 200 to both sides can improve measurement accuracy by calculating an average of measurement results obtained from separate measurements by the respective gas sensor devices 200 disposed at the left and right ears or by calculating and removing a noise component.

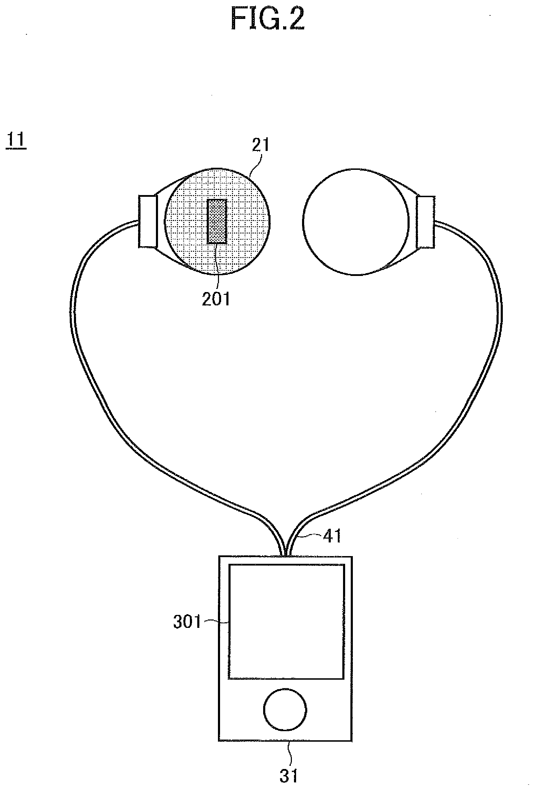

In the embodiment illustrated in FIG. 1, the wearable sound wave conversion device 20 is of a headphone type, but the wearable sound wave conversion device of the present invention is not limited to this and may be any appropriate wearable sound wave conversion device that can form a closed space for accommodating a percutaneous gas around a user's ear. In another embodiment, as illustrated in FIG. 2, a wearable sound wave conversion device 21 may be of an earphone type. In this case, the closed space is formed between the inside of a user's ear and the user contact surface of the earphone. Note that components in a biological gas measurement device 11 other than a wearable sound wave conversion device 21, such as a gas reservoir structure 51, a moisture removal and restraint mechanism 101, a gas sensor device 201, a handheld terminal 31, a cable 41 and a display 301, have the same functions as those illustrated in FIG. 1.

According to the present invention, when a user views and listens to music and a video, the user can obtain and grasp the biological information, typically a user's own health condition, without need of wearing a dedicated device for obtaining and grasping the biological information. For example, by using the biological gas measurement device of the present invention to measure acetone serving as a measure of the burned amount of fat in the body, the user can know what amount of fat in the body has been burned through his or her exercise without the need of wearing a dedicated biological gas measurement device while listening to music by means of the sound wave conversion function provided in the biological gas measurement device. Also, by measuring ammonia serving as a measure of liver disorder and so on, the user can grasp a user's own health problem while listening to music by means of the sound wave conversion function provided in the biological gas measurement device and/or viewing a video on a handheld terminal during user's commuting.

Although the wearable sound wave conversion device, such as a headphone and an earphone, has been described in the above-stated embodiments as typical instances of the wearable electronic device, the present invention is not limited to this and may be any appropriate wearable device that is worn by a user in contact with a user's skin and can measure a biological gas released from a user's skin surface or expiration during wearing. In other words, the wearable device may be an electronic device worn by the user in contact with a user's skin for its intended purpose in daily life. For example, the wearable device may have purposes other than measurement of a biological gas component and may be portable.

Similar to the above-stated wearable sound wave conversion devices 20 and 21, a wearable device includes a gas reservoir structure to form a space for accumulating a biological gas component released from a user's skin surface or expiration and a gas sensor device to measure the biological gas component accumulated in the formed space. Furthermore, the wearable device may include a moisture removal and restraint mechanism to remove or restrain the moisture from/in the space formed with the gas reservoir structure. Also, the moisture removal and restraint mechanism may be activated at any appropriate timing in synchronization with measurement timings of the gas sensor device. In other words, the moisture removal and restraint mechanism may remove or restrain the moisture from/in the space formed with the gas reservoir structure before or at the same time as the measurement timings of the biological gas component by the gas sensor device. In addition, the moisture removal and restraint mechanismmay be activated at any appropriate timing depending on its implementations. For example, if the moisture removal and restraint mechanism is implemented in a humidity absorbent, such as a water absorbent, a dehumidification agent or a desiccant agent, the moisture removal and restraint mechanism may be activated before or at the same time as the measurement timings of the biological gas component by the gas sensor device. Also, if the moisture removal and restraint mechanism is implemented in a miniaturized air blower, the moisture removal and restraint mechanism may be activated before the measurement timings of the biological gas component by the gas sensor device.

Also, the wearable device may be connected to a handheld device carried by a user in a wired or wireless manner. In other words, fundamental structures and operations of components in the wearable device may be the same as those of the above-stated wearable sound wave conversion device.

For example, the wearable device includes a watch worn in contact with a user's arm, a mobile phone and a handheld device worn to a part of a user's body. Also, the wearable device may be a bracelet, a wristband, a ring, a necklace, earrings, a hair band, glasses, a mask, an adhesive plaster, clothing, a hat, gloves, shoes or any other things worn in contact with a user's body if components thereof such as the above-stated gas sensor device do not need an internal power source or can obtain a power source from outside light or others.

According to the above-stated embodiments, a user can obtain and grasp the biological information, typically a user's own health condition in a natural manner in daily life, by only wearing the above-stated wearable electronic device or wearable device without need of carrying a dedicated biological gas detection device.

Although the embodiments of the present invention have been described in detail, the present invention is not limited to the above-stated embodiments, and various variations and modifications can be made within the scope of the present invention as defined by claims.

### LIST OF REFERENCE SYMBOLS

10, 11: biological gas measurement device
20, 21: wearable sound wave conversion device
30, 31: handheld terminal
40, 41: cable
50, 51: gas reservoir structure
100, 101: moisture removal and restraint mechanism
200, 201: gas sensor device
300, 301: display

## Claims

1. A wearable device (20) comprising:
a gas reservoir structure configured to form a space for accumulating a biological gas component released from a skin surface of a wearer; and
a gas sensor device (200) configured to measure the biological gas component accumulated in the formed space,
**characterised in that**
the gas sensor device includes gas sensors disposed at left and right sides of the wearable device, for disposition at the left and right ears of the wearer respectively, the gas sensors being configured to measure different types of percutaneous gas components at the left side and the right side of the wearable device respectively.

2. The wearable device as claimed in claim 1, further comprising:
a moisture removal and restraint mechanism configured to remove or restrain a moisture from/in the formed space including the skin surface of the user.

3. The wearable device as claimed in claim 2, wherein the moisture removal and restraint mechanism comprises a water absorbent, a dehumidification agent, a desiccant agent, a miniaturized air blower or a combination thereof.

4. The wearable device as claimed in claim 1, wherein the wearable device comprises a headphone or an earphone.

5. The wearable device as claimed in claim 1, wherein the wearable device includes a wearable sound wave conversion device configured to output a measurement result measured by the gas sensor device and converted into audio information, the converted measurement result being superimposed onto other audio information.

6. A biological gas measurement device comprising:
a wearable device as claimed in any one of claims 1 to 5; and
a handheld terminal (30) communicatively connected to the wearable device,
wherein the handheld terminal is configured to receive biological information indicative of the biological gas component measured by the wearable device and to provide the user with the biological information.

7. The biological gas measurement device as claimed in claim 6, wherein the handheld terminal (30) is configured to store the biological information in a storage device in the handheld terminal and/or to transmit the biological information to a server connected via a network.

8. A biological gas measurement method, comprising:
measuring, when a user wears a wearable device in contact with the user's skin, by a gas sensor device (200) in the wearable device, a biological gas component accumulated in a space formed between the user and the wearable device;
transmitting, by the wearable device, biological information indicative of the measured biological gas component to a handheld terminal (30) communicatively connected to the wearable device; and
providing, by the handheld terminal, the user with the received biological information,
**characterised in that**
the gas sensor device includes gas sensors disposed at the user's left and right ears when worn, and different types of percutaneous gas components are measured separately at the left side and the right side by the gas sensors.

9. The biological gas measurement method as claimed in claim 8, further comprising:
removing or restraining a moisture from/in the space formed between the user and the wearable device.

10. The biological gas measurement method as claimed in claim 8, further comprising:
converting the biological information into audio information, superimposing the converted biological information onto other audio information, and outputting the converted and superimposed biological information from the wearable device.

## Patentansprüche

1. Am Körper tragbare Vorrichtung (20), die Folgendes umfasst:
eine Gasreservoirstruktur, die dafür konfiguriert ist, einen Raum zum Sammeln eines biologischen Gasbestandteils, der von einer Hautoberfläche eines Trägers freigesetzt wird, zu bilden, und
eine Gassensorvorrichtung (200), die dafür konfiguriert ist, den in dem gebildeten Raum gesammelten biologischen Gasbestandteil zu messen,
**dadurch gekennzeichnet, dass**
die Gassensorvorrichtung Gassensoren einschließt, die an der linken und der rechten Seite der am Körper tragbaren Vorrichtung angeordnet sind, zur Anordnung an dem linken beziehungsweise dem rechten Ohr des Trägers, wobei die Gassensoren dafür konfiguriert sind, unterschiedliche Arten von perkutanen Gasbestandteilen an der linken beziehungsweise der rechten Seite der am Körper tragbaren Vorrichtung zu messen.

2. Am Körper tragbare Vorrichtung nach Anspruch 1, die ferner Folgendes umfasst:
einen Feuchtigkeitsentfernungs- und -rückhaltemechanismus, der dafür konfiguriert ist, eine Feuchtigkeit von/in dem gebildeten Raum einschließlich der Hautoberfläche des Trägers zu entfernen oder zurückzuhalten.

3. Am Körper tragbare Vorrichtung nach Anspruch 2, wobei der Feuchtigkeitsentfernungs- und -rückhaltemechanismus ein Wasserabsorptionsmittel, ein Entfeuchtungsmittel, ein Trockenmittel, ein miniaturisiertes Luftgebläse oder eine Kombination derselben umfasst.

4. Am Körper tragbare Vorrichtung nach Anspruch 1, wobei die am Körper tragbare Vorrichtung einen Kopfhörer oder Ohrhörer umfasst.

5. Am Körper tragbare Vorrichtung nach Anspruch 1, wobei die am Körper tragbare Vorrichtung eine am Körper tragbare Schallwellen-Umwandlungsvorrichtung einschließt, die dafür konfiguriert ist, ein Messergebnis auszugeben, das durch die Gassensorvorrichtung gemessen und zu Audioinformationen umgewandelt wird, wobei das umgewandelte Messergebnis auf andere Audioinformationen überblendet wird.

6. Messvorrichtung für biologisches Gas, die Folgendes umfasst:
eine am Körper tragbare Vorrichtung nach einem der Ansprüche 1 bis 5 und
ein Handendgerät (30), das kommunikativ mit der am Körper tragbaren Vorrichtung verbunden ist,
wobei das Handendgerät dafür konfiguriert ist, biologische Informationen zu empfangen, die den biologischen Gasbestandteil anzeigen, der durch die am Körper tragbare Vorrichtung gemessen wird, und den Benutzer mit den biologischen Informationen zu versorgen.

7. Messvorrichtung für biologisches Gas nach Anspruch 6, wobei das Handendgerät (30) dafür konfiguriert ist, die biologischen Informationen in einem Speichergerät in dem Handendgerät zu speichern und/oder die biologischen Informationen an einen Server zu senden, der über ein Netz verbunden ist.

8. Messverfahren für biologisches Gas, wobei das Verfahren Folgendes umfasst:
Messen, wenn ein Benutzer eine am Körper tragbare Vorrichtung in Berührung mit der Haut des Benutzers trägt, durch eine Gassensorvorrichtung (200) in der am Körper tragbaren Vorrichtung, eines biologischen Gasbestandteils, der in einem Raum gesammelt wird, der zwischen dem Benutzer am Körper tragbaren Vorrichtung gebildet wird, und
Senden, durch die am Körper tragbare Vorrichtung, von biologischen Informationen, die den gemessenen biologischen Gasbestandteil anzeigen, an ein Handendgerät (30), das kommunikativ mit der am Körper tragbaren Vorrichtung verbunden ist, und
Versorgen, durch das Handendgerät, des Benutzers mit den biologischen Informationen,
**dadurch gekennzeichnet, dass**
die Gassensorvorrichtung Gassensoren einschließt, die an dem linken beziehungsweise dem rechten Ohr des Trägers angeordnet sind, wenn sie getragen wird, und unterschiedliche Arten von perkutanen Gasbestandteilen gesondert an der linken Seite und der rechten Seite durch die Gassensoren gemessen werden.

9. Messverfahren für biologisches Gas nach Anspruch 8, das ferner Folgendes umfasst:
Entfernen oder Zurückhalten einer Feuchtigkeit von/in dem Raum, der zwischen dem Benutzer und der am Körper tragbaren Vorrichtung gebildet wird.

10. Messverfahren für biologisches Gas nach Anspruch 8, das ferner Folgendes umfasst:
Umwandeln der biologischen Informationen zu Audioinformationen, Überblenden der umgewandelten biologischen Informationen auf andere Audioinformationen und Ausgeben der umgewandelten und überblendeten biologischen Informationen aus der am Körper tragbaren Vorrichtung.

## Revendications

1. Dispositif pouvant être porté (20) comprenant :
une structure de réservoir de gaz conçue pour former un espace pour accumuler un composant gazeux biologique exhalé à partir d'une surface de la peau d'un porteur, et
un dispositif de capteur de gaz (200) conçu pour mesurer le composant gazeux biologique accumulé dans l'espace formé,
**caractérisé en ce que**
le dispositif de capteur de gaz comprend des capteurs de gaz disposés à des côtés gauche et droit du dispositif pouvant être porté, destinés respectivement à être disposés au niveau des oreilles gauche et droite du porteur, les capteurs de gaz étant conçus pour mesurer différents types de composants gazeux percutanés respectivement au niveau du côté gauche et du côté droit du dispositif pouvant être porté.

2. Dispositif pouvant être porté selon la revendication 1, comprenant en outre :
un mécanisme d'enlèvement et de rétention d'humidité conçu pour enlever ou retenir l'humidité de / dans l'espace formé incluant la surface de la peau de l'utilisateur.

3. Dispositif pouvant être porté selon la revendication 2, dans lequel le mécanisme d'enlèvement et de rétention de l'humidité comprend un produit absorbant l'eau, un agent de déshumidification, un agent siccatif, un ventilateur d'air miniaturisé ou une combinaison de ceux-ci.

4. Dispositif pouvant être porté selon la revendication 1, qui comprend un casque ou des écouteurs.

5. Dispositif pouvant être porté selon la revendication 1, selon lequel le dispositif pouvant être porté inclut un dispositif de conversion en onde sonore pouvant être porté et conçu pour délivrer un résultat de mesure mesuré par le dispositif de capteur de gaz et converti en une information sonore, le résultat de mesure converti étant superposé sur une autre information sonore.

6. Dispositif de mesure de gaz biologique comprenant :
un dispositif pouvant être porté selon l'une quelconque des revendications 1 à 5, et
un terminal portatif (30) relié en communication au dispositif pouvant être porté,
dans lequel le terminal portatif est conçu pour recevoir une information biologique représentative du composant gazeux biologique mesuré par le dispositif pouvant être porté et pour fournir à l'utilisateur l'information biologique.

7. Dispositif de mesure de gaz biologique selon la revendication 6, dans lequel le terminal portatif (30) est conçu pour stocker l'information biologique dans un dispositif de mémoire dans le terminal portatif et/ou pour transmettre l'information biologique vers un serveur connecté par l'intermédiaire d'un réseau.

8. Procédé de mesure de gaz biologique, comprenant les opérations suivantes :
mesure, lorsqu'un utilisateur porte un dispositif pouvant être porté en contact avec sa peau, par un dispositif de capteur de gaz (200) dans le dispositif pouvant être porté, d'un composant gazeux biologique accumulé dans un espace formé entre l'utilisateur et le dispositif pouvant être porté,
transmission, par le dispositif pouvant être porté, d'une information biologique représentative du composant gazeux biologique mesuré vers un terminal portatif (30) relié en communication au dispositif pouvant être porté, et
fourniture, par le terminal portatif, de l'information biologique reçue à l'utilisateur,
**caractérisé en ce que**
le capteur de gaz comprend des capteurs de gaz disposés sur les oreilles gauche et droite de l'utilisateur lorsque le dispositif est porté, et différents types de composants gazeux percutanés sont mesurés séparément sur les côtés gauche et droit par les capteurs de gaz.

9. Procédé de mesure de gaz biologique selon la revendication 8, comprenant en outre :
l'enlèvement ou la rétention de l'humidité de/dans l'espace formé entre l'utilisateur et le dispositif pouvant être porté.

10. Procédé de mesure de gaz biologique selon la revendication 8, comprenant en outre :
la conversion de l'information biologique en une information sonore, la superposition de l'information biologique convertie sur une autre information sonore et la délivrance de l'information biologique convertie et superposée depuis le dispositif pouvant être porté.
